# EUROPEAN PATENT SPECIFICATION

(11) **EP 0 881 896 B1**
(45) Date of publication and mention of the grant of the patent: **02.05.2002**
(21) Application number: 97902254.8
(22) Date of filing: 23.01.1997
(51) Int. Cl.: A61K 7/48, A61K 31/60

(54) **SKIN TREATMENT WITH SALICYLIC ACID ESTERS AND RETINOIDS**
HAUTBEHANDLUNGSMITTEL MIT SALICYLSÄUREESTERN UND RETINOIDEN
TRAITEMENT DE LA PEAU AVEC DES ESTERS DE L'ACIDE SALICYLIQUE ET DES RETINOIDES

(30) Priority: 23.02.1996 US 12217 P
(43) Date of publication of application: 09.12.1998
(73) Proprietor: FD MANAGEMENT, INC., Wilmington, Delaware 19801 (US)
(72) Inventor: COREY, Joseph, Michael, Waterbury, CT 06706 (US); GUERRERO, Angel, Augusto, Huntington, CT 06484 (US)
(74) Representative: Gervasi, Gemma, Dr.
(86) International application number: EP9700387
(87) International publication number: WO9730691

(56) References cited:
- EP-A- 0 676 194
- EP-A- 0 713 696

## Description

### BACKGROUND OF THE INVENTION

### Field of the Invention

The present invention concerns cosmetic compositions containing certain esters of salicylic acid and retinoids and methods of treating skin with such compositions.

### The Related Art

Skin is subject to deterioration through dermatologic disorders or normal aging (chronoaging) as well as extrinsic factors (environmental). Dermatologic disorders include such conditions as acne, dry skin, dandruff, keratosis, pruritus, inflammatory dermatoses, eczema, psoriasis and tenia pedis (athlete's foot).

Chronoaging results in the thinning and general degradation of skin. As skin naturally ages, there is reduction in the cells and blood vessels that supply the skin. There is also a flattening of the dermal-epidermal junction which results in weaker mechanical resistance. Older individuals increasingly develop facial fine lines, wrinkles, leatheriness, yellowing, sagging, mottling (hyperpigmentation), age spots and the general signs of aging.

Extrinsic factors are primarily those caused by exposure to sun. Changes are most prominent in light skinned individuals who burn easily and tan poorly. The results of photodamage may be identical to those of aging except appearing at an accelerated rate. Wrinkling, yellowing, leatheriness, mottling and hyperpigmentation are all associated with sun damage. Most disturbing to many individuals is the wrinkling effect. It is a prime reminder of the disappearance of youth. As a result, there have been many reports of cosmetic treatments aimed at the elimination of wrinkles.

PCT applications WO 93/10755 and WO 93/10756 report salicylic acid as an effective anti-wrinkling agent. U.S. Patent 5,262,407 reports use of ring acylated salicylic acid as a treatment against skin aging. Salicylic acid has also been described for the treatment of acne in U.S. Patent 4,891,227 and U.S. Patent 4,891,228. Moreover, salicylic acid has been used for the removal of warts, corns and calluses; for the treatment of psoriasis, seborrheic dermatitis and dandruff; and for the topical treatment of ringworm infection. A listing of commercially available products containing salicylic acid will be found in the Physician's Desk Reference, 45th Edition, 1991, page 323.

Ring alkylated salicylic acid has been reported in Japanese Patent 4036238 (Takasago Perfumery KK) for treatment of acne vulgaris.

Significant irritation is often associated with the use of salicylic acid. Another problem is that salicylic acid is not always readily formulatable into oily compositions. Derivatives of salicylic acid most often leave the acidic function free. Irritation caused by acidity is therefore not prevented by such derivatives.

Skin care compositions containing retinoids have also become quite prominent in recent years. Retinoic acid, also known as Vitamin A acid or Tretinoin, is well known for treatment of acne. Even more recently, the retinoids have been suggested as treatment against photoaging and sun damage. For instance, U.S. Patent 4,603,146 discloses Vitamin A acid in an emollient vehicle to prevent skin aging. U.S. Patent 4,877,805 suggests a number of retinoids as useful for restoring and reversing sun damage in human skin. EP 0 631 772 describes use of retinol in combination with an irritation ameliorating amount of glycolic acid.

EP 0 676 194 describes cosmetic compositions containing a cocktail of an alpha-hydroxy acid, a salicylic acid or ester thereof and a retinoid. While various dermatological benefits are claimed for the cocktail, there is a need for further improvements.

Accordingly it is an object of the present invention to provide a treatment for a variety of dermatologic disorders such as acne, dry skin, dandruff, keratosis, pruritus, inflammatory dermatosis, eczema, psoriasis and tinea pedis.

Another object of the present invention is to provide a treatment for chronoaging conditions including wrinkling and fine lines, leatheriness, yellowing, sagging, mottling (hyperpigmentation), age spots and the general signs of aging.

Still another object of the present invention is to provide a treatment against environmental abuse to skin including wrinkling and fine lines, yellowing, leatheriness, mottling and hyperpigmentation.

Yet another object of the present invention is to provide a treatment to improve the condition of skin with a composition and active that does not impart irritation.

These and other objects of the present invention will become more readily apparent from the following summary and detailed discussion.

### SUMMARY OF THE INVENTION

A cosmetic composition is provided including:
(i) a safe and effective amount of a salicylate ester having formula (I): 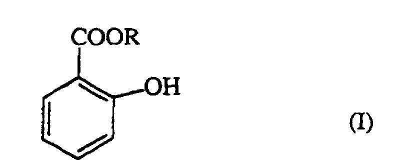 wherein R is a C₁₁-C₃₀ alkyl or alkenyl radical;
(ii) a safe and effective amount of retinol or a C₁₈-C₃₀ fatty acid ester thereof; and
(iii) a safe and effective amount of a pharmaceutically acceptable carrier.

A method is also provided for treating skin conditions selected from the group consisting of dermatologic skin disorders, chronoaging, environmental abuse and combinations thereof, by applying to the skin a composition including as an active a combination of retinol or a C₁₈-C₃₀ fatty ester thereof and a salicylate ester having the structure (I) : 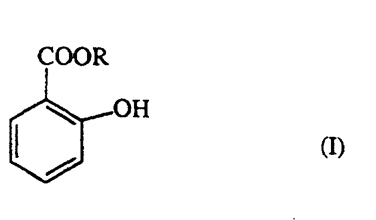 wherein R is a C₁₁-C₃₀ alkyl or alkenyl radical.

### DETAILED DESCRIPTION OF THE INVENTION

Now it has been discovered that deterioration of skin through dermatologic disorders, chronoaging and environmental abuse (e.g. sun and wind) can be reduced, inhibited and even reversed through application of a cosmetic composition including as active a combination of retinol or a C₁₈-C₃₀ fatty acid ester thereof and a non-ring ester derivative of salicylic acid having formula (I): 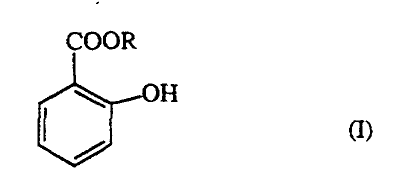 wherein R is a C₁₁-C₃₀ alkyl or alkenyl radical. Most preferred are the C₁₂-C₂₀, optimally the C₁₃ esters of salicylic acid.

Retinol or its C₁₈-C₃₀ fatty acid ester is one of the two actives essential for the method and compositions of the present invention. Vitamin A esters such as Vitamin A palmitate or acetate are not here effective and do not fall within the scope of this invention. Retinol esters which are effective include retinyl linoleate, retinyl linoleneate, retinyl oleate, retinyl stearate, retinyl behenate and mixtures thereof. Most preferred is retinyl linoleate.

"Safe and effective amounts" of the C₁₁-C₃₀ esters of salicylic acids and retinol and its esters are to be used within cosmetic compositions of the present invention. The term "safe and effective amounts" are defined as any amount sufficient to significantly induce a positive modification in the condition to be treated, but low enough to avoid serious side effects (at a reasonable benefit/risk ratio), within the scope of sound medical judgement. The safe and effective amount of the salicylate esters will vary with the particular condition being treated, the age and physical condition of the person being treated, the severity of the condition, the duration of the treatment, the nature of concurrent treatment, the specific ester employed, the particular pharmaceutically-acceptable carrier utilized, and like factors in the knowledge and expertise of the attending specialist. Generally the amounts of salicylate ester may range from 0.01 to 20%, preferably from 0.1 to 10%, more preferably from 1 to 8%, optimally from 2 to 6% by weight of the composition. Amounts of retinol or its esters may range from 0.01 to 10%, preferably from 0.1 to 5%, more preferably from 0.2 to 0.5% by weight of the composition.

Besides the retinol or ester thereof and salicylate ester, compositions of the present invention will utilize a pharmaceutically acceptable carrier. The carrier may either be aqueous, anhydrous or an emulsion. Preferably the compositions are aqueous, especially water and oil emulsions of the W/O or O/W variety. Water when present will usually be in amounts which may range from 5 to 95%, preferably from 20 to 70%, optimally between 35 and 60% by weight of the composition.

Besides water, relatively volatile solvents may also serve as carriers within compositions of the present invention. Most preferred are monohydric C₁-C₃ alkanols. These include ethyl alcohol, methyl alcohol and isopropyl alcohol. The amount of monohydric alkanol may range from 1 to 70%, preferably from 10 to 50%, optimally between 25 to 40% by weight.

Emollient materials may also serve as pharmaceutically acceptable carriers. These may be in the form of silicone oils and synthetic esters. Amounts of the emollients may range anywhere from 0.1 to 30%, preferably between 1 and 20% by weight.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms.

Linear volatile silicone materials generally have viscosities less than about 5 centistokes at 25°C while cyclic materials typically have viscosities of less than about 10 centistokes.

Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 100,000 centistokes at 25°C. Among the preferred non-volatile emollients useful in the present compositions are the polydimethyl siloxanes having viscosities from about 10 to about 400 centistokes at 25°C.

Among the ester emollients are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl'monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate.
(5) Sterols esters, of which cholesterol fatty acid esters are examples thereof.

Fatty acids having from 10 to 30 carbon atoms may also be included as pharmaceutically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be employed as pharmaceutically acceptable carriers in compositions of this invention. The humectant aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol. The amount of humectant may range anywhere from 0.5 to 30%, preferably between 1 and 15% by weight of the composition.

Thickeners may also be utilized as part of the pharmaceutically acceptable carrier of compositions according to the present invention. Typical thickeners include crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenum, pectin and combinations of these gums. Amounts of the thickener may range from 0.0001 to 5%, usually from 0.001 to 1%, optimally from 0.01 to 0.5% by weight.

Collectively the water, solvents, silicones, esters, fatty acids, humectants and/or thickeners will constitute the pharmaceutically acceptable carrier in amounts from 1 to 99.9%, preferably from 80 to 99% by weight.

Cosmetic compositions of the present invention may be in any form. These forms may include lotions, creams, roll-on formulations, mousses, aerosol and non-aerosol sprays and pad-applied formulations.

Surfactants may also be present in cosmetic compositions of the present invention. Total concentration of the surfactant, if present, will typically range from 0.1 to 40%, preferably from 1 to 20%, optimally from 1 to 5% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates and combinations thereof.

Sunscreen actives may also be included in compositions of the present invention. Particularly preferred are such materials as ethylhexyl p-methoxycinnamate, available as Parsol MCX, and benzophenone-3, also known as Oxybenzone. Inorganic sunscreen actives may be employed such as microfine titanium dioxide, polyethylene and various other polymers. Amounts of the sunscreen agents will generally range from 0.1 to 30%, preferably from 2 to 20%, optimally from 4 to 10% by weight.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may also contain water-soluble vitamins. The term water-soluble defines substances with a solubility of at least 0.1%, preferably at least 1%, optimally at least 5% by weight in water. Illustrative water-soluble vitamins are Niacin, Vitamin B₂, Vitamin B₆, Vitamin C and Biotin. One source for Vitamin C is a product sold under the trademark of Vitazyme C available from the Brooks Company. Niacin, Vitamin B and Biotin are available from Roche Pharmaceuticals. Total amount of vitamins in compositions according to the present invention may range from 0.001 to 1%, preferably from 0.01 to 0.6, optimally from 0.1 to 0.5% by weight.

Keratolytic agents such as C₂-C₂₅ α-hydroxy alkanoic acids may also be incorporated into compositions of this invention. Illustrative of this group of materials are glycolic, lactic, α-hydroxyoctanoic acids and salts thereof. The salts may be selected from alkalimetal, ammonium and C₁-C₂₀ alkyl or alkanolammonium counterions. Levels of α-hydroxyalkanoic acids may range from 0.001 to 10%, preferably between 0.2 and 1%, optimally between 0.4 and 0.5% by weight.

Another adjunct ingredient can be that of an enzyme. Particularly preferred is superoxide dismutase, commercially available as Biocell SOD from the Brooks Company, USA.

Natural vegetable materials from renewable resources are often desirable in cosmetic compositions. For instance, cosmetic compositions of the present invention may include β-glucan derived from oats, commercially available under the trademark Microat SF from Nurture Inc., Missoula, Montana.

Colorants, fragrances, opacifiers and abrasives may also be included in compositions of the present invention.

Each of these substances may range from about 0.05 to about 5%, preferably between 0.1 and 3% by weight.

The following Examples will more fully illustrate embodiments of this invention. All parts, percentages and proportions referred to herein and in the appended claims are by weight unless otherwise indicated.

### EXAMPLE 1

A skin cream formulation of the oil in water type according to the present invention is described in Table I.

**TABLE I**

| **CHEMICAL** | **% W/W** |
|---|---|
| Water | qs |
| Disodium EDTA | 0.10 |
| Polysorbate 40 | 1.00 |
| Propylene Glycol | 3.00 |
| Methylparaben | 0.25 |
| Tridecyl Salicylate | 5.00 |
| Retinol | 0.30 |
| Isopropyl Palmitate | 5.00 |
| Isostearyl Isostearate | 3.00 |
| Silicone 200/100 Fluid (Dimethicone) | 5.00 |
| Silicone 344 Fluid (Cyclomethicone) | 15.00 |
| Imidazolidinyl Urea | 0.20 |
| Sepigel 305 (Laureth-7, Polyacrylamide, Isoparaffin) | 3.00 |

### EXAMPLE 2

Another skin cream formulation of the oil in water type according to the present invention is described in Table II.

**TABLE II**

| **CHEMICAL** | **% W/W** |
|---|---|
| water | qs |
| Disodium EDTA | 0.10 |
| Butylene Glycol | 3.00 |
| Glycerin | 3.00 |
| Methylparaben | 0.25 |
| Tridecyl Salicylate | 5.00 |
| Retinyl Linoleate | 0.30 |
| Squalane | 1.00 |
| Shea Butter | 0.50 |
| Cetyl Alcohol | 1.50 |
| Octyl Palmitate | 2.00 |
| C12-C15 Alkyl Benzoate | 5.00 |
| Octyl Stearate | 2.00 |
| Silicone 344 Fluid (Cyclomethicone) | 2.00 |
| Imidazolidinyl Urea | 0.20 |
| Sepigel 305 (Laureth-7, Polyacrylamide, Isoparaffin) | 3.50 |

### EXAMPLE 3

Still another skin cream formulation of the oil in water type according to the present invention is described in Table III.

**TABLE III**

| **CHEMICAL** | **% W/W** |
|---|---|
| Water | qs |
| Disodium EDTA | 0.10 |
| Butylene Glycol | 2.00 |
| Glycerin | 3.00 |
| Methylparaben | 0.25 |
| Tridecyl Salicylate | 5.00 |
| Retinol | 0.30 |
| Shea Butter | 0.50 |
| Cetyl Alcohol | 1.00 |
| PEG-100 Glycerol Monostearate | 4.00 |
| C12-15 Alkyl Benzoate | 6.00 |
| Tocopheryl Linoleate | 0.50 |
| Silicone 200 Fluid (Dimethicone) | 2.00 |
| Imidazolidinyl Urea | 0.20 |

### EXAMPLE 4

A microemulsion formulation according to the present invention is described in Table IV.

**TABLE IV**

| **CHEMICAL** | **% W/W** |
|---|---|
| PPG-5-Ceteth-20 | 4.00 |
| PEG-40 Hydrogenated Castor Oil | 1.75 |
| Polyglyceryl-10 Decaoleate | 10.00 |
| PEG-8 Caprylic/Capric Glycerides | 10.00 |
| SDA Alcohol 40B | 12.00 |
| Isodecyl Neopentanoate | 16.00 |
| Glyceryl Trioctanoate | 8.00 |
| Cyclomethicone (DC 344 Fluid) | 8.00 |
| Propylparaben | 0.10 |
| Isostearic Acid | 2.50 |
| Tridecyl Salicylate | 5.00 |
| Retinyl Behenate | 0.30 |
| Phenoxyethanol | 0.30 |
| Deionized Water | qs |

### EXAMPLE 5

A skin cream formulation of the water in oil type according to the present invention is described in Table V.

**TABLE V**

| **CHEMICAL** | **% W/W** |
|---|---|
| Cyclomethicone (DC 344 Fluid) | 12.00 |
| Dimethicone (DC 200/10 fluid) | 2.00 |
| Dimethicone Copolyol | 2.50 |
| Cetyl Dimethicone | 0.50 |
| C12-15 Alkyl Benzoate | 3.00 |
| Tridecyl Salicylate | 5.00 |
| Retinyl Linoleate | 0.10 |
| Glycerin | 3.00 |
| Propylene Glycol | 2.00 |
| Disodium EDTA | 0.10 |
| Methylparaben | 0.25 |
| Sodium Chloride | 1.20 |
| Phenoxyethanol | 0.20 |
| Deionized Water | qs |

### EXAMPLE 6

An anhydrous serum formulation according to the present invention is described in Table VI.

**TABLE VI**

| **CHEMICAL** | **% W/W** |
|---|---|
| Sepigel 305 | 1.50 |
| SD Alcohol 40 B (200 proof) | 20.00 |
| Cyclomethicone (DC 344 Fluid) | 2.50 |
| Squalene | 1.00 |
| Octyl Isononanoate | 2.50 |
| Dimethicone (DC 200 Fluid | 5.20 |
| Isononyl Isononanoate | 30.00 |
| Butylene Glycol | 1.00 |
| Propylparaben | 0.10 |
| Tridecyl Salicylate | 5.00 |
| Retinol | 0.50 |
| Dimethiconol | 2.75 |

### EXAMPLE 7

A sunscreen lotion formulation of the oil in water type according to the present invention is described in Table VII.

**TABLE VII**

| **CHEMICAL** | **% W/W** |
|---|---|
| Water | qs |
| Disodium EDTA | 0.10 |
| Butylene Glycol | 2.00 |
| Glycerin | 5.00 |
| Methylparaben | 0.25 |
| Tridecyl Salicylate | 5.00 |
| Retinyl Linoleneate | 0.10 |
| Octyl Methoxycinnamate | 7.50 |
| Benzophenone-3 | 2.50 |
| Shea Butter | 0.50 |
| Cetyl Alcohol | 1.50 |
| Octyl Palmitate | 2.00 |
| C12-15 Alkyl Bezoate | 2.00 |
| Silicone 200 Fluid (Dimethicone) | 1.00 |
| Imidazolidinyl Urea | 0.20 |
| Sepigel 305 (Laureth-7, Polyacrylamide, Isoparaffin) | 3.50 |

### EXAMPLE 8

Dermatologic disorders, chronoaging and environmental abuse all have some relationship to sebum production. The present example reports an in vitro analysis of sebum suppression by use of a combination of salicylate ester and retinoid.

### In Vitro sebocyte Lipogenesis Assay

Human sebaceous glands were isolated from the nose of a male (age 60) and cultured using submerged tissue culture techniques (Bajor et al, J. Invest, Dermatol, 102:1994. P. 564). These sebocytes accumulate intracellular lipid droplets characteristic of mature human sebum.

Harvested and passaged sebocytes were added to each well of a 48 well tissue culture plate and incubated at 37°C in the presence of 7.5% CO₂ for 14 days. The growth medium was changed three times per week. On the day of experimentation, the growth medium was removed and the sebocytes washed three times with phosphate buffered saline (PBS). Fresh PBS in 0.5 ml amount was added to each well and 10 microliters of active agent speculated to inhibit lipogenesis. Triplicate wells were utilized for each sample. Controls consisted of PBS, dimethyl sulfoxide (DMSO) used to solubilize the lipophilic compounds, and phenol red, a compound which possesses estrogen-like activity. The cultures were incubated at 37°C/7.5% CO₂ for 30 minutes. Radioactive label was prepared by adding 100 ul of 14-C labelled acetic acid (Amersham, sodium salt, specific activity of 56 mCi/mmol) to 10 mL of 50 mM sodium acetate buffer. Then 50 ul was added to each well containing the sebocytes and active agents. The cultures were returned to the incubator for 4 hours. Thereafter the treatments and label were removed and the sebocytes rinsed three times with fresh PBS. Sebocytes were harvested and 10 microliters removed and set aside for protein assessment. The remaining samples containing the 14-C label were extracted and the label counted using a Beckman scintillation counter. Triplicates were performed for each sample.

For each 48 well tissue culture plate, 16 samples could be analyzed. Of these, 1 sample was reserved for PBS, 1 sample for DMSO, and 1 sample for phenol red leaving 13 remaining samples. When TDS (tridecyl salicylate) was analyzed in the presence of other compounds, an additional set of wells was used for measuring the effects of TDS alone. When multiple 48 well plates were used, TDS was included in each plate.

**TABLE VIII**

| **Retinyl Linoleate Concentration** | **Retinyl Linoleate % Actual Reduction (SD)** | **Retinyl Linoleate + 10 uM TDS % Actual Reduction (SD)** | **Retinyl Linoleate +10 uM TDS Theoretical (Additive) Reduction** |
|---|---|---|---|
| 100 nM | 4.4 (2.9) | 21.1 (10.8) | 24.1 |
| 1 uM | 6.0 (19.5) | 26.1 (8.8) | 25.7 |
| 10 uM | 13.8 (7.6) | 50.0 (2.1) | 33.5 |
| 100 uM | 20.3 (3.3) | 41.5 (6.7) | 40.0 |
| **10 uM TDS = 19.7% Reduction** | | | |

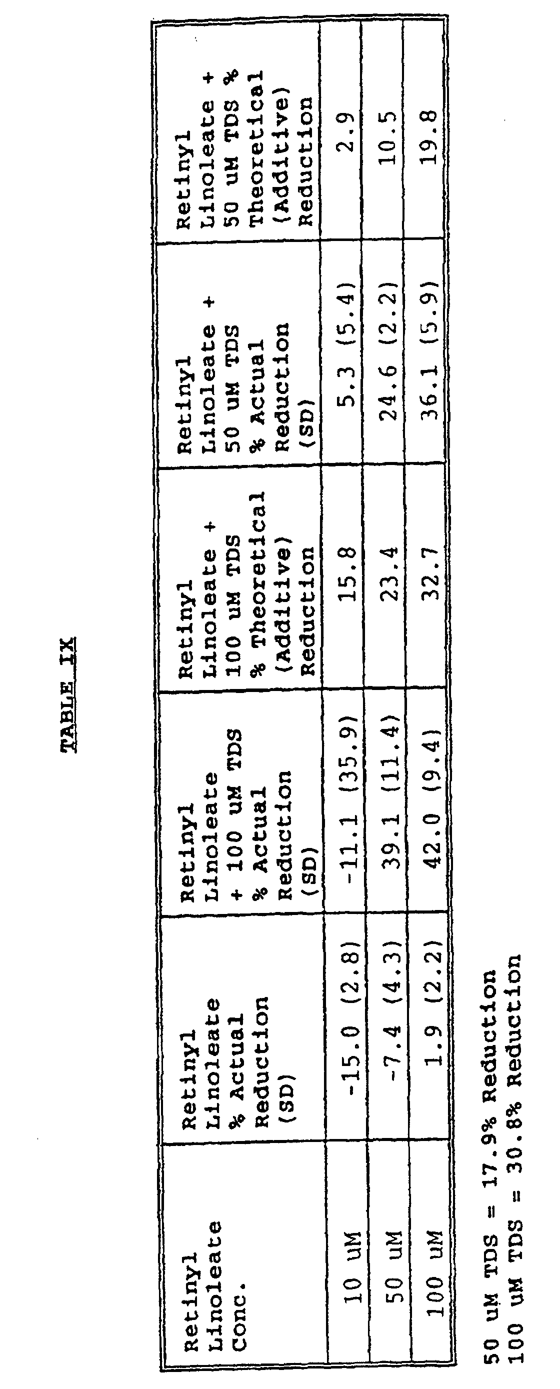

**TABLE X**

| **Retinyl Palmitate Concentration** | **Retinyl Palmitate % Actual Reduction (SD)** | **Retinyl Palmitate + 100 uM TDS % Actual Reduction (SD)** | **Retinyl Palmitate + 100 uM TDS % Theoretical (Additive) Reduction** |
|---|---|---|---|
| 10 uM | 12.2 (6.4) | 51.8 (7.1) | 50.9 |
| 100 uM | 12.0 (13.3) | 51.8 (5.2) | 50.7 |
| **100 uM TDS = 38.7% Reduction** | | | |

From the foregoing Tables it is evident that retinyl linoleate appears to have a synergistic activity with tridecyl salicylate. By contrast, retinyl palmitate exhibited only an additive effect. Similar trials were done using retinol instead of retinyllinoleate. Retinol was at very low levels synergistic with tridecyl salicylate.

## Claims

1. A cosmetic composition comprising:
(i) a safe and effective amount of a salicylate ester having formula (I): 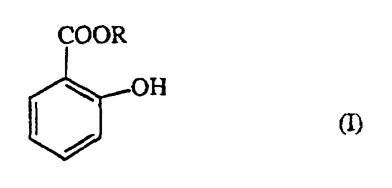 wherein R is a C₁₁-C₃₀ alkyl or alkenyl radical;
(ii) a safe and effective amount of retinol or a C₁₈-C₃₀ fatty acid ester thereof; and
(iii) a safe and effective amount of a pharmaceutically acceptable carrier.

2. The cosmetic composition according to claim 1 wherein the salicylate ester is a C₁₂-C₂₀ alkyl or alkenyl ester of salicylic acid.

3. The cosmetic composition according to claim 1 wherein the salicylate ester is tridecyl salicylate.

4. The cosmetic composition according to any one of claims 1-3 wherein the retinol fatty acid ester is retinyl linoleate.

5. A cosmetic method for treating skin conditions selected from the group consisting of dermatologic disorders, chronoaging and environmental abuse, the method comprising applying to the skin a safe and effective amount of a combination of retinol or a C₁₈-C₃₀ fatty acid ester thereof and a salicylate ester, the salicylate ester having the formula (I) : 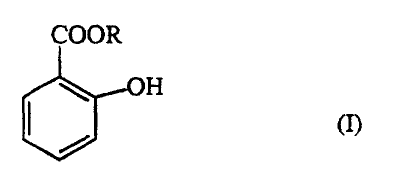 wherein R is a C₁₁-C₃₀ alkyl or alkenyl radical.

6. A method according to claim 5 wherein the dermatologic disorders are selected from the group consisting of acne, dry skin, dandruff, keratosis, pruritus, inflammatory dermatitis, eczema, psoriasis and tinea pedis.

7. A method according to claim 5 wherein the chronoaging is **characterized by** a condition selected from the group consisting of wrinkling, fine lines, leatheriness, yellowing, sagging, hyperpigmentation, age spots and general signs of aging.

8. A method according to claim 5 wherein environmental abuse includes conditions selected from the group consisting of wrinkling, fine lines, leatheriness, yellowing, mottling and hyperpigmentation.

9. A method according to any one of claims 5-8 wherein R is a C₁₂-C₂₀ alkyl or alkenyl group.

10. A method according to any one of claims 5-8 wherein the salicylate ester is tridecyl salicylate.

11. A method according to any one of claims 5-10 wherein the retinol fatty acid ester is retinyl linoleate.

## Patentansprüche

1. Kosmetische Zusammensetzung umfassend:
(i) eine sichere und wirksame Menge an Salicylatester mit der Formel (I) : 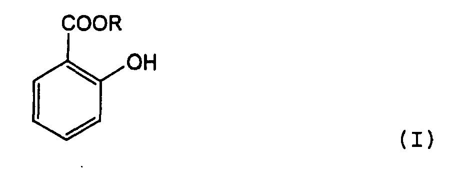 wobei R ein C₁₁-C₃₀ Alkyl- oder Alkenylradikal ist;
(ii) eine sichere und wirksame Menge Retinol oder einem C₁₈-C₃₀ Fettsäureester davon; und
(iii) eine sichere und wirksame Menge eines pharmazeutisch annehmbaren Trägers.

2. Kosmetische Zusammensetzung gemäß Anspruch 1, wobei der Salicylatester ein C₁₂-C₂₀ Alkyl- oder Alkenylester der Salicylsäure ist.

3. Kosmetische Zusammensetzung gemäß Anspruch 1, wobei der Salicylatester Tridecylsalicylat ist.

4. Kosmetische Zusammensetzung gemäß einem der Ansprüche 1 bis 3, wobei der Retinolfettsäureester Retinyllinoleat ist.

5. Kosmetisches Verfahren zur Behandlung von Hautzuständen, ausgewählt aus der Gruppe bestehend aus dermatologischen Erkrankungen, Alterserscheinungen und schädlichen Umwelteinflüssen, wobei die Methode das Auftragen einer sicheren und wirksamen Menge einer Mischung aus Retinol oder einem C₁₈-C₃₀ Fettsäureester davon und einem Salicylatester auf die Haut umfaßt, wobei der Salicylatester die Formel (I) hat: 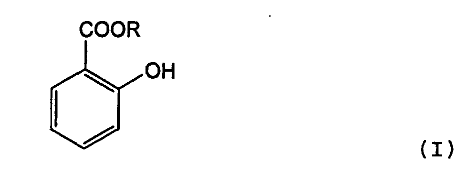 wobei R ein C11-C30 Alkyl- oder Alkenylradikal ist.

6. Verfahren gemäß Anspruch 5, wobei die dermatologischen Erkrankungen aus der Gruppe bestehend aus Akne, trockener Haut, Schuppen, Keratose, Hautjucken, entzündlicher Dermatitis, Ekzemen, Psoriasis und Tinea pedis ausgewählt werden.

7. Verfahren gemäß Anspruch 5, wobei die Alterserscheinungen durch einen Zustand charakterisiert sind, der aus der Gruppe, die aus Faltenbildung, feinen Linien, Lederartigkeit, Vergilbung, Erschlaffung, Hyperpigmentierung, Altersflecken und allgemeinen Anzeichen des Alterns besteht, ausgewählt wird.

8. Verfahren gemäß Anspruch 5, wobei die schädlichen Umwelteinflüsse Bedingungen einschließen, die aus der Gruppe bestehend aus Faltenbildung, feinen Linien, Lederartigkeit, Vergilbung, Fleckenbildung und Hyperpigmentierung ausgewählt werden.

9. Verfahren gemäß einem der Ansprüche 5 bis 8, wobei R eine C₁₂-C₂₀ Alkyl- oder Alkenylgruppe ist.

10. Verfahren gemäß einem der Ansprüche 5 bis 8, wobei der Salicylatester Tridecylsalicylat ist.

11. Verfahren gemäß einem der Ansprüche 5 bis 10, wobei der Retinolfettsäureester Retinyllinoleat ist.

## Revendications

1. Composition cosmétique comprenant :
(i) une quantité sûre et efficace d'un salicylate ayant la formule (I) 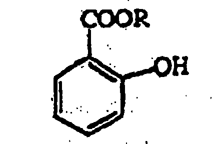 dans laquelle R est un radical alkyle ou alcényle en C₁₁-C₃₀
(ii) une quantité sûre et efficace de rrétinol ou d'un de ses esters d'acide gras en C₁₈-C₃₀ ; et
(iii) une quantité sûre et efficace d'un support pharmaceutiquement acceptable.

2. La composition cosmétique selon la revendication 1, dans laquelle le salicylate est un ester alkylé ou alcénylé en C₁₂-C₂₀ d'acide salicylique.

3. La composition cosmétique selon la revendication 1, dans laquelle le salicylate est le salicylate de tridécyle.

4. La composition cosmétique selon l'une qualconque des revendications 1-3, dans laquelle l'ester d'acide gras de rétinol est le linoléate de rétinyle.

5. Méthode de traitement cosmétique d'affections cutanées choisies dans le groupe constitué par les affections dermatologiques, le vieillissement et les agressions de l'environnement, ladite méthode comprenant l'application sur la peau d'une quantité sûre et efficace d'une combinaison de rétinol ou d'un de ses esters d'acide gras en C₁₈-C₃₀ et d'un salicylate, le salicylate ayant la formule (I) : 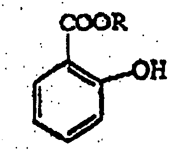 dans laquelle R est un radical alkyle ou alcényle en C₁₁-C₃₀.

6. Méthode selon la revendication 5, dans laquelle les affections dermatologiques sont choisies dans le groupe constitué par l'acné, la peau sèche, les pellicules, la kératose, le prurit, la dermite inflammatoire, l'eczéma, le psoriasis et le tinea pedis.

7. Méthode selon la revendication 5, dans laquelle le vieillissement est **caractérisé par** une affection choisie dans le groupe constitué par les rides, les ridules, le durcissement, le jaunissement, le relâchement, l'hyperpigmentation, les taches de vieillesse, et les signes généraux de vieillissement.

8. Méthode selon la revendication 5, dans laquelle les agressions de l'environnement comprennent les affections choisies dans le groupe constitué par les rides, les ridules, le durcissement, le jaunissement, les marbrures et l'hyperpigmentation.

9. Méthode selon l'une quelconque des revendications 5-8, dans laquelle R est un radical alkyle ou alcényle en C₁₂-C₂₀.

10. Méthode selon l'une quelconque des revendications 5-8, dans laquelle le salicylate est le salicylate de tridécyle.

11. Méthode selon l'une quelconque des revendications 5-10, dans laquelle l'ester d'acide gras de rétinol est le linoléate de rétinyle.
